# EUROPEAN PATENT APPLICATION

(11) **EP 1 757 294 A1**
(43) Date of publication of application: **28.02.2007**
(21) Application number: 05758953.3
(22) Date of filing: 14.06.2005
(51) Int. Cl.: A61K 31/519

(54) **ORALLY-ADMINISTERED AQUEOUS RISPERIDONE SOLUTION**

(30) Priority: 15.06.2004 ES 200401456
(71) Applicant: Farmalider, S.A., E-28100 Alcobendas (Madrid) (ES)
(72) Inventor: JIMENEZ REDONDO, Ana, E-28108 Alcobendas (ES); SANZ MENENDEZ, Nuria, E-28108 Alcobendas Madrid) (ES); GOMEZ CALVO, Antonia, E-28108 Alcobendas (Madrid) (ES); MARTINEZ ALZAMORA, Fernando, E-28108 Alcobendas (Madrid) (ES); MUÑOZ RUIZ, Angel, E-28108 Alcobendas (Madrid) (ES)
(74) Representative: Gil-Vega, Victor
(86) International application number: PCT/ES2005/000336
(87) International publication number: WO 2005/123085

(57) **Abstract**

The invention relates to an orally-administered aqueous risperidone solution comprising a buffer system which maintains the pH of the solution at values of between 6 and 7.5 and one more preservative agents which are effective in said pH interval. The aqueous risperodine solution, which can optionally contain other excipients such as appetising, sweetening and/or flavouring agents, remains stable for at least 12 months at 25° C and 60% HR and 6 months at 40° C and 75% HR.

## Description

### Technical field

The present invention relates to aqueous risperidone solutions for oral administration which have high stability during their storage.

### Prior art

Risperidone is the common international name corresponding to the compound 3-[2-[4-(6-fluoro-1,2-benzisoxazol-3-yl)-1-piperidinyl]ethyl]-6,7,8,9-tetrahydro-2-methyl-4H-pyrido[1,2-a] pyrimidin-4-one, whose structural formula is:

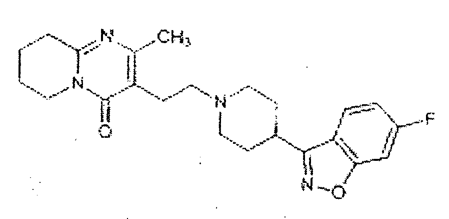

Risperidone is used in medicine as an antipsychotic agent.

European patent application EP 0196132-A1 discloses the preparation of risperidone and other analogous products, and in its example 11 also discloses a composition for oral solution that consists of an unbuffered solution which contains sorbitol and glycerine, a relatively small quantity of water and preservatives.

Patent application PCT WO 96/01652-A1 explains that the composition for oral solution disclosed in patent application EP 0196132-A1 has stability problems due to interactions of sorbitol with the drug substance risperidone, which may also, possibly, interact with other polyhydroxylated alcohols similar to sorbitol.

The technical solution proposed in patent application WO 96/01652-A1 to resolve the problem arising consists of a composition for oral solution which consists of an aqueous risperidone solution that is substantially free from sorbitol and which comprises a buffering system capable of keeping the solution pH between 2 and 6, preferably between 3 and 4.

This limitation in the pH value implies a barrier which restricts the development of alternative formulations of oral risperidone solutions, which suggests the need of having alternative technical solutions which enable the person skilled in the art to have available a wider range of formulation possibilities.

Surprisingly, the authors of the present invention have discovered that risperidone compositions for oral solutions can be formulated, in the form of aqueous solutions substantially free from sorbitol, which have a high stability despite being formulated in pH ranges over 6.

### Object of the invention

The object of the present invention is an aqueous risperidone solution for oral administration, substantially free from sorbitol, whose pH is over 6.0, despite which it has high stability during its storage for prolonged periods, without reducing its efficacy and bioavailability.

### Detailed description of the invention

The aqueous risperidone solution for oral administration object of the invention is substantially free from sorbitol and is characterized in that it comprises:
- a buffering system selected from the citric acid/ disodium phosphate, disodium phosphate/monosodium phosphate and monopotassium phosphate/sodium hydroxide group, adjusted so that the solution pH is between 6.0 and 7.5, and
- one or more preservatives which are effective in the pH range between 6.0 and 7.5.

It should be understood that the term risperidone used in this description comprises risperidone in the form of free base, the addition salts thereof with pharmaceutically acceptable acids and/or its solvates.

The risperidone content in the aqueous solution for oral administration may be between 0.02 and 0.5%, expressed by weight of free base risperidone by solution volume, being especially preferred a content of 0.1 % (w/v), which corresponds to 1 mg of free base risperidone for each ml of solution.

The citric acid/disodium phosphate, disodium phosphate/monosodium phosphate and monopotassium phosphate/sodium hydroxide buffering systems are well known by the person skilled in the art, and the adjustment of its components to attain the desired pH range is very easy for the person skilled in the art by routine tests, being, furthermore, explained in numerous basic consultation manuals.

It should be understood that when this description refers to disodium phosphate, monosodium phosphate and monopotassium phosphate, said expressions are abbreviated references to, respectively, disodium monohydrogen phosphate, monosodium dihydrogen phosphate and monopotassium dihydrogen phosphate.

Preferably, the selected buffering system is citric acid/disodium phosphate.

Preferably, the buffering system is adjusted so that the aqueous risperidone solution object of the invention has a pH value between 6.3 and 7.0.

PH ranges over 7.5 should be avoided, since above said pH risperidone may become partly insoluble and cause precipitations.

The preservative or preservatives comprised in the oral solution object of the invention, whose function is to avoid microorganism growth, is/are selected so that it/they does/do not react with the drug substance or other excipients and which has a water solubility which avoids its precipitation. It is especially necessary for the purposes of the present invention that the preservative or preservatives maintain their antimicrobial efficacy in the pH range between 6.0 and 7.5.

The commercial catalogues on preservatives usually specify their chemical incompatibilities and their efficacy in accordance with different pH ranges, for which reason it is no problem for the person skilled in the art to select the suitable preservatives for the purposes of the present invention.

Preferably, the preservative is selected from the group domiphen bromide, methylparaben, ethylparaben, propylparaben and butylparaben, and/or their mixtures, both in the form of acid and salt. The alkylparaben derivatives can be used alone or in combination to produce greater antimicrobial efficacy. Said derivatives are commercially available in acid form or in the form of salts, for which reason they can be used in any of these presentations.

In an especially preferred form, the preservative selected is domiphen bromide, which in the antimicrobial efficacy tests performed has shown excellent results at low concentrations.

The concentration of the preservative agent can be between 0.005% and 0.5% by weight/volume.

When the preservative agent selected is domiphen bromide a concentration between 0.005% and 0.2% by weight/volume can be used.

The preservative efficacy present in oral solution, object of the invention, has been evaluated using the method described in the European Pharmacopoeia 4th Ed. 2003, according to which the solution is contaminated with high concentrations of microorganisms ("Challenge Test"). This test determines if the preservative is affected by the storage conditions, the drug substance or other components of the formulation.

The test performed on a pH 7.0 oral buffered solution containing 0.009% (w/v) domiphen bromide, reveals that said preservative is effective in preserving the oral risperidone solution, object of the invention, against possible microorganism contaminations.

The aqueous solution for oral administration object of the invention may further contain, optionally, other excipients well known in pharmaceutical technology. Among them we can mention, for example, flavourings such as sodium chloride, artificial sweeteners such as aspartame, cyclamate, saccharin, potassium acesulfame, and/or aromas. The compositions object of the invention are substantially free from sorbitol or other similar polyhydroxylated alcohols.

An especially preferred pH 6.4 buffered aqueous solution comprises the following components:
- 1.0 mg/ml of risperidone
- 0.10 mg/ml of domiphen bromide.
- 5.96 mg/ml of anhydrous citric acid, and
- 49.35 mg/ml of disodium phosphate dodecahydrate

Alternatively, a pH 7.0 buffered aqueous solution, also especially preferred, comprises the following components:
- 1.0 mg of risperidone
- 0.10 mg/ml of domiphen bromide.
- 3.41 mg/ml of anhydrous citric acid, and
- 58.88 mg/ml of disodium phosphate dodecahydrate

Surprisingly, the aqueous risperidone solutions object of the invention, despite being formulated in a pH range between 6.0 and 7.5 are maintained stable during long storage periods, for example during 12 months at a temperature of 25°C and a relative humidity of 60%, and under accelerated conditions during 6 months at a temperature of 40°C and a relative humidity of 75%. After this time period, the appearance of the solution is correct, the pH only has variations of around 1% or less, and the risperidone and preservative agent content are kept substantially unaltered.

The data regarding stability of oral risperidone solutions according to the present invention, in different temperature and relative humidity conditions, are shown in the Examples section of this description.

Aqueous risperidone solutions, object of the invention are suitable for their dilution with water or another non-alcoholic drink, except tea, in order to facilitate their administration to the patient.

The following examples are given for the purposes of providing the person skilled in the art with a detailed explanation of specific embodiments within the invention and the benefits obtained therewith.

### Example 1 Preparation of a pH 7.0 buffered aqueous risperidone solution.

In a stainless steel reactor equipped with a stirrer, place 450 ml of purified water, add 4.50 g of sodium chloride (flavouring agent) and keep stirring until the complete dissolution of the solid.

Add 0.50 mg of aspartame (sweetening agent) to the solution produced and keep stirring until its total dissolution.

Then add 0.050 g of domiphen bromide (preservative agent) and keep stirring until its dissolution.

In the previous solution, disperse 0.50 g of risperidone and 0.25 g of lemon aroma and stir until complete homogenisation.

Then, add 1.70 g of anhydrous citric acid and stir until total dissolution.

Next, incorporate 29.44 g of disodium phosphate dodecahydrate and keep stirring until complete dissolution.

Finally, flush with water until a volume of 500 ml.

The aqueous risperidone solution produced is transparent, slightly yellowish and with a characteristic lemon aroma. The initial pH observed is 7.0±0.1.

The results of the stability test of the oral solution prepared is shown in Table I.

**TABLE 1**

| Time/temperature /RH | Appearance | pH | Risperidone (%) | Preservative (%) |
|---|---|---|---|---|
| Start | Correct | 6.98 | 100.71 | 103.84 |
| 6 months 25°C/60% | Correct | 6.92 | 99.53 | 102.34 |
| 6 months 30°C/65% | Correct | 6.92 | 103.23 | 100.93 |
| 6 months 40°C/75% | Correct | 6.93 | 98.26 | 100.03 |
| 12 months 25°C/60% HR | Correct | 6.92 | 99.43 | 104.40 |

| | | | | |
|---|---|---|---|---|
| (RH = relative humidity) | | | | |

The determinations of the risperidone and domiphen bromide content are performed using standard HPLC chromatography techniques, using suitable standards.

It can be observed that the pH 7.0 buffered solution shows excellent stability with regard to its appearance, pH, drug substance and preservative, even at least during 12 months at 25°C and 60% RH, and under accelerated conditions during 6 months at a temperature of 40°C and a relative humidity of 75%.

### Example 2 Preparation of a pH 6.4 buffered aqueous risperidone solution

Following the procedure described in Example 1, prepare 500 ml of a pH 6.4 buffered aqueous risperidone solution. In this case, the buffering system comprises 2.98 of anhydrous citric acid and 24.67 of disodium phosphate.

The aqueous risperidone solution produced is transparent, slightly yellowish and with a characteristic lemon aroma. The initial pH observed is 6.4 ± 0.1.

The results of the stability test of the oral solution prepared is shown in Table II.

**TABLE 1**

| Time/temperature /RH | Appearance | pH | Risperidone (%) | Preservative (%) |
|---|---|---|---|---|
| Start | Correct | 6.34 | 101.94 | 100.80 |
| 6 months 25°C/60% RH | Correct | 6.27 | 100.45 | 102.71 |
| 6 months 30°C/65% RH | Correct | 6.28 | 99.02 | 101.79 |
| 6 months 40°C/75% RH | Correct | 6.27 | 102.02 | 102.26 |
| 12 months 25°C/60% RH | Correct | 6.29 | 101.03 | 102.66 |

| | | | | |
|---|---|---|---|---|
| (RH = relative humidity) | | | | |

It can be observed that the pH 6.4 buffered solution shows excellent stability with regard to its appearance, pH, drug substance and preservative, even at least during 12 months at 25°C and 60% RH, and under accelerated conditions during 6 months at a temperature of 40°C and a relative humidity of 75%.

## Claims

1. Aqueous risperidone solution for oral administration substantially free from sorbitol, **characterized in that** it comprises:
- a buffering system selected from the citric acid/ disodium phosphate, disodium phosphate/monosodium phosphate and monopotassium phosphate/sodium hydroxide group, adjusted so that the solution pH is between 6.0 and 7.5, and
- one or more preservatives which are effective in the pH range between 6.0 and 7.5.

2. Aqueous solution according to claim 1, **characterized in that** the buffering system consists of citric acid and disodium phosphate.

3. Aqueous solution according to claims 1 to 2, **characterized in that** it has a pH value between 6.3 and 7.0.

4. Aqueous solution according to claims 1 to 3, **characterized in that** the preservative concentration is between 0.005% and 0.5% by weight/volume.

5. Aqueous solution according to claims 1 to 4, **characterized in that** the preservative agent is selected from the group domiphen bromide, methylparaben, ethylparaben, propylparaben and butylparaben, and/or their mixtures.

6. Aqueous solution according to claim 5, **characterized in that** the preservative agent is domiphen bromide.

7. Aqueous solution according to claim 6, **characterized in that** the domiphen bromide concentration is between 0.0005% and 0.2% by weight/volume.

8. Aqueous solution according to claims 1 to 7, **characterized in that** the risperidone content is between 0.02 and 0.05% expressed by weight of free base risperidone by solution volume.

9. Aqueous solution according to claim 8, **characterized in that** the risperidone content corresponds to 1 mg of free base risperidone for each ml of solution.

10. Aqueous risperidone solution for oral administration which comprises:
- 1.0 mg/ml of risperidone
- 0.10 mg/ml of domiphen bromide.
- 5.96 mg/ml of anhydrous citric acid, and
- 49.35 mg/ml of disodium phosphate dodecahydrate

11. Aqueous risperidone solution for oral administration which comprises
- 1.0 mg of risperidone
- 0.10 mg/ml of domiphen bromide.
- 3.41 mg/ml of anhydrous citric acid, and
- 58.88 mg/ml of disodium phosphate dodecahydrate
